# EUROPEAN PATENT SPECIFICATION

(11) **EP 1 736 184 B1**
(45) Date of publication and mention of the grant of the patent: **26.02.2020**
(21) Application number: 05727440.9
(22) Date of filing: 29.03.2005
(51) Int. Cl.: A61M 25/10, A61M 1/10, A61M 25/00

(54) **BALLOON CATHETER**
BALLONKATHETER
CATHETER A BALLON

(30) Priority: 31.03.2004 JP 2004103299; 31.03.2004 JP 2004103306
(43) Date of publication of application: 27.12.2006
(73) Proprietor: Zeon Corporation, Tokyo 100-8246 (JP)
(72) Inventor: MORI, Kenji, c/o ZEON MEDICAL INC., Tokyo 1050011 (JP); IIDA, Takahiro, c/o ZEON MEDICAL INC., Tokyo 1050011 (JP)
(74) Representative: Maiwald Patent- und Rechtsanwaltsgesellschaft mbH
(86) International application number: PCT/JP2005/005820
(87) International publication number: WO 2005/094917

(56) References cited:
- JP-A- 3 051 059
- JP-A- 5 123 403
- JP-A- 8 215 314
- JP-A- 10 118 187
- JP-A- 2000 107 293
- JP-A- 2001 346 883
- JP-A- 2003 000 701
- US-A- 5 711 754
- US-B1- 6 447 479
- US-B1- 6 503 223

## Description

### TECHNICAL FIELD

The present invention relates to a balloon catheter, more particularly relates to a balloon catheter suitably used for the intra-aortic balloon pumping method - a method of treatment for example acute heart failure etc.

### BACKGROUND ART

As a circulatory support method for support of heart functions by inserting a balloon catheter in the aorta and inflating and deflating the balloon in accordance with the heart beat for treatment at the time of deterioration of heart functions, the intra-aortic balloon pumping (IABP) method is known.

A variety of performances are sought for the intra-aortic balloon catheter used for the IABP method. Among these, a thin outside diameter is being sought for preventing lower limb ischemia, reducing the burden on the patient, and other reasons. Further, in recent years, to further reduce the patient burden, the insertion of the catheter from the brachial artery instead of the conventional femur artery has been investigated, so an intra-aortic balloon catheter having an even thinner outside diameter is being sought.

However, if simply making a catheter thinner, the flow resistance of the channel through which the fluid circulates for the inflation and deflation of the balloon is increased, the time required for the inflation and deflation of the balloon increases (response deteriorates), and the inflation and deflation of the balloon in accordance with the heart beat become difficult. For this reason, improvement of the response has become necessary for reducing the diameter of an intra-aortic balloon catheter.

As a technique for improving the response of this intra-aortic balloon catheter, the technique of fastening the inner tube to the inside wall of the outer tube by adhesion, fusion, or other means is known (JP H05 123403 A: Document 1). According to this technique, the increase of the flow resistance arising due to twisting of the inner tube in the outer tube is prevented and, as a result, the response is improved.

However, if driving an intra-aortic balloon catheter using this technique over a long period of time, the phenomenon of the response gradually becoming poorer may arise. In the worst case, the balloon can no longer be inflated and deflated in accordance with the heart beat and the effect of heart function assistance ends up falling drastically in some cases.

By the way, JP 2003 000701 A (Document 2) describes an intra-aortic balloon catheter designed to prevent the phenomenon of the inner tube and outer tube moving relative to each other and making insertion of the catheter into the body harder, by providing a separate member for engagement at the inside of the distal end of the outer tube and fastening the inner tube and outer tube near the distal end of the outer tube.

However, with the method of fastening the outer tube and inner tube only near the distal end of the outer tube like as the balloon catheter described in this Document 2, the majority of the inner tube is not fastened to the outer tube, so the inner tube become twisted in the outer tube and the response become poor in some cases.

Further, with the technique of fastening the inner tube and outer tube by inserting a separate member for fastening inside the outer tube like as the balloon catheter of JP 2003 000701 A, because that separate member exists, the distal end opening face of the outer tube becomes narrower. Because of this, the flow resistance of the fluid for the inflation and deflation of the balloon increased at the inserted part of the separate member and the time required for the inflation and deflation of the balloon increased (response became poorer) in some cases. If the response of the balloon becomes poor, inflation and deflation of the balloon in accordance with the heart beat become difficult, and the effect of heart function assistance ends up falling.

Note that in an intra-aortic balloon catheter such as described in Document 1 where the inner tube and outer tube are fastened along the majority of the total length of the outer tube by adhesion or fusion, the adhesion or fusion sufficiently prevents relative movement of the inner tube and outer tube, so it had been thought that there was absolutely no necessity of providing a separate member for engagement as described in Document 2.

Further, in the intra-aortic balloon catheter described in JP 2000 005318 A (Document 3), the inner tube and the outer tube are fastened near the distal end of the outer tube for the purpose of preventing the phenomenon of relative movement of inner tube and outer tube and the resultant harder insertion of the catheter into body.

However, with the method of adhesion of the outer tube and inner tube by an adhesive only near the distal end of the outer tube like as the balloon catheter of Document 3, securing a sufficient bonding area is difficult and sufficient bonding strength is not obtained. For this reason, the bonded location sometimes ended up peeling apart during insertion of the balloon catheter.

US 5 711 754 A relates to a balloon catheter having an inner tube communicating with a blood introduction portion provided at the tip of the balloon portion. The inner tube is affixed to the inner wall of the catheter tube by adhesion, melt-bonding, or integral formation. Further disclosed is a balloon catheter having the balloon portions at a range of the end sides of the balloon portions smaller in a sectional area compared with the balloon portions at the tip sides.

US 6 447 479 B1 relates to a blood vessel dilatation apparatus including an inner tube having a lumen; an outer tube forming a lumen between the outer tube and an outer surface of the inner tube; a dilatation member; a first opening provided at a rear end portion of the inner tube and communicating with the lumen; a second opening provided at a rear end portion of the outer tube and communicating with the lumen. The outer tube includes a front-side tube and a rear-side tube fixed to the front-side tube. The front-side tube has a diameter-changing portion whose outer and inner diameters decrease toward its front end. A front portion of the rear-side tube is connected with an inner surface a rear end portion of the front-side tube. An inner surface of the front portion of the rear-side tube is connected with the inner tube.

### PROBLEM TO BE SOLVED BY THE INVENTION

The present invention was made in consideration of this situation. A first object is to provide an intra-aortic balloon catheter which does not cause an increase in the patient burden,. reduces the flow resistance of the pressurized fluid conduit, inflates and deflates the balloon part with a good response, and maintains excellent response even when driven for a long period of time.

Further, a second object of the present invention is to provide a balloon catheter that uses simple means to strongly fasten the inner tube and outer tube near the distal end of the outer tube and has a large opening area at the distal end of the outer tube and can inflate and deflate the balloon part with a good response.

### MEANS FOR SOLVING THE PROBLEM

The invention is defined by the appended claims.

The inventors engaged in intensive studies to achieve the first object and as a result discovered that in an intra-aortic balloon catheter that fastens the outer tube and inner tube through adhesion or fusion, driving the balloon for a long time will cause the outer tube and inner tube to peel apart gradually from the near the distal end of the outer tube causing a worsening of the response along with time. Also, they discovered that by fastening the outer tube and inner tube of an intra-aortic balloon catheter by adhesion or fusion and further fastening the outer tube and inner tube by engagement near the distal end of the outer tube, the peeling of the inner tube and outer tube by long time driving will be prevented and, as a result, deterioration of the response over time is prevented. Based on this discovery, they completed the present invention.

Thus, the intra-aortic balloon catheter according to a first aspect of the present invention provides an intra-aortic balloon catheter comprising;
a balloon part inserted inside the aorta and inflating and deflating to assist the heart function,
an outer tube with a distal end connected to a proximal end of the balloon part to introduce and guide out pressurized fluid inside of the balloon part, and
an inner tube to which a distal end of the balloon part is connected and extending through the insides of the balloon part and the outer tube in the axial direction, characterized in that
the inner tube is fused or adhered with the inside wall of the outer tube over a length of 50% or greater of the total length from the distal end of the outer tube and in that
an engagement means engages the inner tube with the inside wall of the outer tube at the distal end of the outer tube.

Preferably, the distal end of the outer tube is formed with a cut that extends in the circumferential direction of the outer tube at a position a predetermined distance away from the distal end opening face of the outer tube toward the proximal end,
an engagement hole enabling insertion of the inner tube is formed by part of the wall of the outer tube positioned in the distance from the cut to the distal end opening face, constituting a cut piece, being pushed in toward the inside of the outer tube, and
the inner tube is inserted in the engagement hole for engagement with the inside wall of the outer tube.

Preferably, the distal end opening face of the outer tube forms an acute angle with a longitudinal axis of the outer tube.

Preferably, the engagement hole is formed with the cut piece positioned so that it protrudes from a distal end opening face of the outer tube to the distal end side.

Preferably, the outside diameter of the inner tube positioned at the distal end side from the engagement means is larger than the outside diameter of the inner tube positioned at the proximal end side from the engagement means.

In the balloon catheter according to the first aspect of the present invention, the outer tube and inner tube are fastened by adhesion or fusion over a length of 50% or greater of the total length of the outer tube. Further, the outer tube and the inner tube are fastened by engagement near the distal end of the outer tube.

In the intra-aortic balloon catheter of the present invention, since the outer tube and inner tube are fastened over a length of 50% or greater of the total length of the outer tube, twisting of the inner tube within the outer tube ceases and an increase in the flow resistance within the outer tube (pressurized fluid conduit) through which the pressurized fluid is circulated is prevented. As a result, the response of the balloon part is improved.

The outer tube and inner tube are preferably fastened over a length of 70% or greater of the total length of the outer tube, still more preferably are fastened over the total length of the outer tube.

Note that this fastening may be any one that substantially prevents the twisting of the inner tube within the outer tube. The fastening need not be continuous over the entire range of the predetermined length. It may also be fastening at predetermined intervals.

The fastening of the outer tube and the inner tube over a length of 50% or greater of the total length of the outer tube is performed by adhesion or fusion. Fastening by adhesion or fusion makes it possible to select the material of the outer tube and inner tube with comparative freedom and there is almost no increase in the flow resistance in the outer tube. In particular, it is preferable to perform the adhesion by fastening from the viewpoint of the work efficiency and a higher degree of freedom of material selection. As opposed to this, if performing this fastening by engagement, the engagement means is liable to cause the flow resistance within the outer tube to increase and the response of the balloon part to deteriorate. Further, if integrally forming the inner tube and outer tube, the range of selection of materials of the inner tube and outer tube is narrowed and selection of preferable materials for each becomes difficult.

The type of adhesive used when fastening by adhesion is not particularly limited. A cyanoacrylate-based adhesive, epoxy-based adhesive, or other adhesive can be used. A cyanoacrylate-based adhesive is particularly preferable.

Further, if fastening by fusion, heat fusion, fusion through solvents, ultrasonic fusion, high frequency fusion, or other techniques can be employed.

In the intra-aortic balloon catheter of the present invention, the outer tube and inner tube are fastened by adhesion or fusion over a length of 50% or greater of the total length of the outer tube and, further, are fastened by engagement at the distal end of the outer tube. This fastening by engagement enables the phenomenon of long time driving of the intra-aortic balloon catheter causing the outer tube and inner tube fastened by adhesion or fusion to peel apart and the response to deteriorate over time to be prevented.

Further, this fastening by engagement need only be performed over a very small range of the distal end of the outer tube, so the increase of the flow resistance within the outer tube due to the engagement means can be kept at a minimum.

The engagement means used for the engagement may be formed by processing the outer tube or inner tube itself or may be made a separate member from the inner tube and outer tube, however, from the viewpoint of the work efficiency and prevention of detachment of the members, it is preferable to form the engagement means by processing the outer tube or the inner tube itself. In particular, processing of the outer tube is preferable.

According to the first aspect of the present invention, there is provided an intra-aortic balloon catheter which does not increase the patient burden, reduces the flow resistance of the pressurized fluid conduit, inflates and deflates the balloon part with a good response, and maintains excellent response even when driven over a long period of time.

To achieve the second object, the balloon catheter according to a second aspect of present invention provides a balloon catheter comprising:
a balloon part able to be inflated and deflated,
an outer tube with a distal end connected to a proximal end of the balloon part so as to introduce and guide out pressurized fluid inside of the balloon part, and
an inner tube to which a distal end of the balloon part is connected extending along the insides of the balloon part and outer tube in the axial direction, characterized in that
the distal end of the outer tube is formed with a cut that extends in the circumferential direction of the outer tube at a predetermined distance away from the distal end opening face of the outer tube toward the proximal end,
an engagement hole enabling insertion of the inner tube is formed by part of the wall of the outer tube positioned in the distance from the cut to the distal end opening face, constituting a cut piece, being pushed in toward the inside of the outer tube, and
the inner tube is inserted in the engagement hole for engagement with the inside wall of the outer tube.

Preferably, the distal end opening face of the outer tube forms an acute angle with a longitudinal axis of the outer tube.

Preferably, the engagement hole is formed with the cut piece positioned so that it protrudes from a distal end opening face of the outer tube to the distal end side.

Preferably, the outside diameter of the inner tube positioned at the distal end side from the engagement means is larger than the outside diameter of the inner tube positioned at the proximal end side from the engagement means.

The balloon catheter according to the second aspect of the present invention preferably is used for the intra-aortic balloon pumping method, but it is also possible to use it as other balloon catheters.

According to the second aspect of the present invention, there is provided a balloon catheter that uses a simple means to strongly fasten the inner tube and outer tube near the distal end of the outer tube, has a large opening area at the distal end of the outer tube, and can inflate and deflate the balloon part with a good response.

### BRIEF DESCRIPTION OF THE DRAWINGS

[FIG. 1] FIG. 1 is a schematic cross-sectional view of an intra-aortic balloon catheter according to a first embodiment of the present invention.
[FIG. 2] FIG. 2 is an enlarged view of details of the engagement part of the outer tube and inner tube shown in FIG. 1.
[FIG. 3] FIG. 3 is a schematic perspective view of the fastening state between outer tube and inner tube shown in FIG. 1.
[FIG. 4] FIG. 4 is a schematic view of a step of making a cut in the distal end of the outer tube.
[FIG. 5] FIG. 5 is a schematic view of a step after FIG. 4.
[FIG. 6] FIG. 6 is a basic cross-sectional view along the line VI-VI in FIG. 5.

### BEST MODE FOR WORKING THE INVENTION

Below, the present invention will be explained based on the embodiments shown in the figures.

### First Embodiment

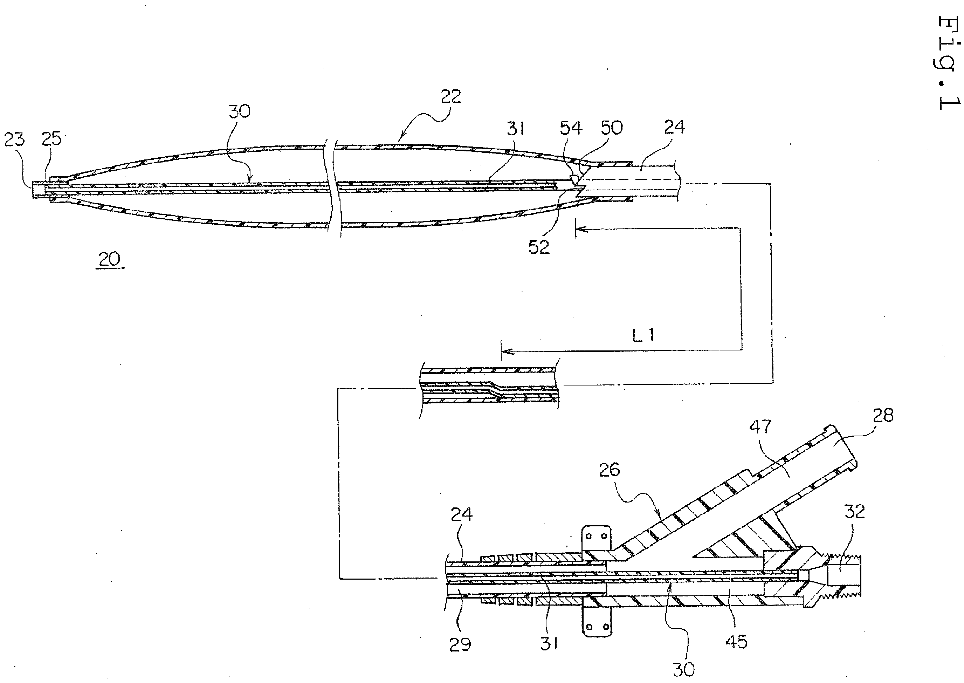

The intra-aortic balloon catheter 20 according to an embodiment of the present invention shown in FIG. 1 is a balloon catheter used for the intra-aortic balloon pumping method and has a balloon part 22 which inflates and deflates in accordance with the heart beat. The balloon part 22 is comprised of a thin film with a thickness of 50 to 150 µm. The thin film is not particularly limited in material, but a material excellent in flex fatigue resistance is preferable, for example, it is comprised of polyurethane. The outside diameter and length of balloon part 22 are determined in accordance with the inside capacity of the balloon part 22, which has a large influence on effect of assistance of heart functions, and the inside diameter of the arterial vessel. The inside capacity of the balloon part 22 is not particularly limited, but may be 20 to 50 cc. The outside diameter of the balloon part 22 is, during inflation, preferably 12 to 16 mm, and the length is preferably 150 to 250 mm.

The distal end of this balloon part 22 is attached to a front tip 25 formed with a blood circulation hole 23 by heat fusion or adhesion or another means. At the inner circumference of the front tip 25, the distal end of the inner tube 30 is attached by heat fusion or adhesion or another means.

The proximal end of the balloon part 22 is connected to the distal end of the outer tube 24. Pressurized fluid is introduced and guided out inside of the balloon part 22 through a pressurized fluid conduit 29 formed inside this outer tube 24, whereby the balloon part 22 inflates and deflates. The balloon part 22 and outer tube 24 are connected by heat fusion or adhesion by an adhesive.

The inner tube 30 extends through the insides of the balloon part 22 and outer tube 24 in the axial direction, is formed inside it with a blood conduit 31 not in communication with the inside of the balloon part 22 and pressurized fluid conduit 29 formed inside in the outer tube 24, and communicates with a blood pressure measurement port 32 of the later explained branching part 26 at the proximal end. The inner tube 30, as is later explained, sends the blood taken in from a blood circulation hole 23 of the front tip 25 to the blood pressure measurement port 32 of the branching part 26. The fluctuation in blood pressure is measured from there.

The inner tube 30 positioned within the balloon part 22 has the deflated balloon part 22 wrapped around it when the balloon catheter 20 is inserted into the artery. The blood conduit 31 can also be used as a tube for insertion of a guide wire used for efficient insertion of the balloon part 22 into the artery.

The proximal end of the outer tube 24 has a branching part 26 joined to it. The branching part 26 is formed separate from the outer tube 24 and is joined with the outer tube 24 by heat fusion or adhesion or another means . The branching part 26 is formed with a first passage 47 having a pressurized fluid inlet/outlet port 28 for introducing and guiding pressurized fluid in a pressurized fluid conduit 29 of the outer tube 24 and the balloon part 22, and with a second passage 45 having a blood pressure measurement port 32 communicating with the blood conduit 31 of the inner tube 30.

The pressurized fluid inlet/outlet port 28 is connected to a non-illustrated pump device. This pump device is used to introduce and guide out the pressurized fluid in the balloon part 22. The pressurized fluid is not particularly limited, but to enable the balloon part 22 to inflate and deflate fast in accordance with the drive of the pump device, one with a low viscosity and mass such as helium gas is used.

The blood pressure measurement port 32 is connected to a non-illustrated blood pressure measuring device to enable the fluctuation of the pressure of the blood in the artery taken from the blood circulation hole 23 near the distal end of the balloon part 22 to be measured. Based on the fluctuation of blood pressure measured by this blood pressure measuring device, the pump device is controlled in accordance with the heart beat to inflate and deflate the balloon part 22 at a short cycle of 0.4 to 1 second.

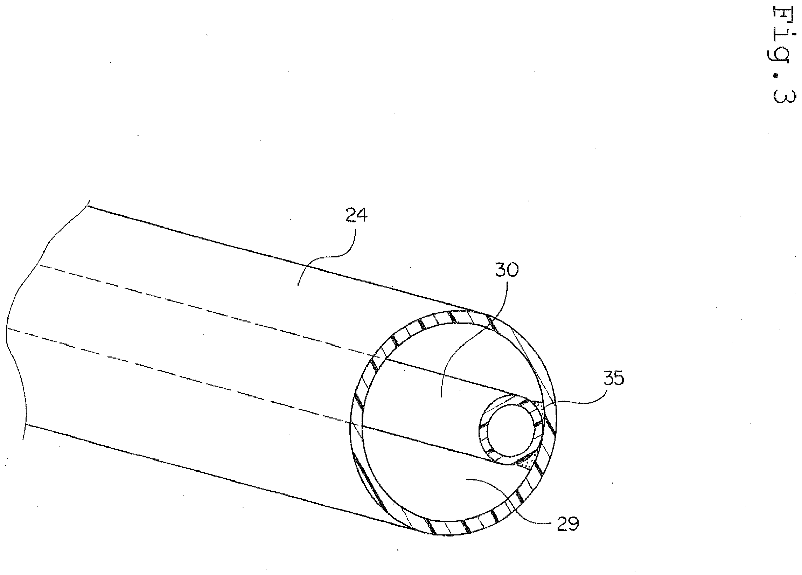

In the present embodiment, as shown in FIG. 1 and FIG. 3, the outer tube 24 and the inner tube 30 are fastened by an adhesive 35 over a length L1 of 50% or greater, preferably 70% or greater, of the total length of the outer tube 24 from the distal end of the outer tube 24. By fastening the outer tube 24 and inner tube 30 in this way, the flow resistance of the pressurized fluid conduit 29 in the outer tube 24 falls and the response of the balloon part 22 are improved. The adhesive 35 used for the fastening is not particularly limited, but a cyanoacrylate-based adhesive, epoxy-based adhesive, or other adhesive can be used, preferably a cyanoacrylate-based adhesive is used.

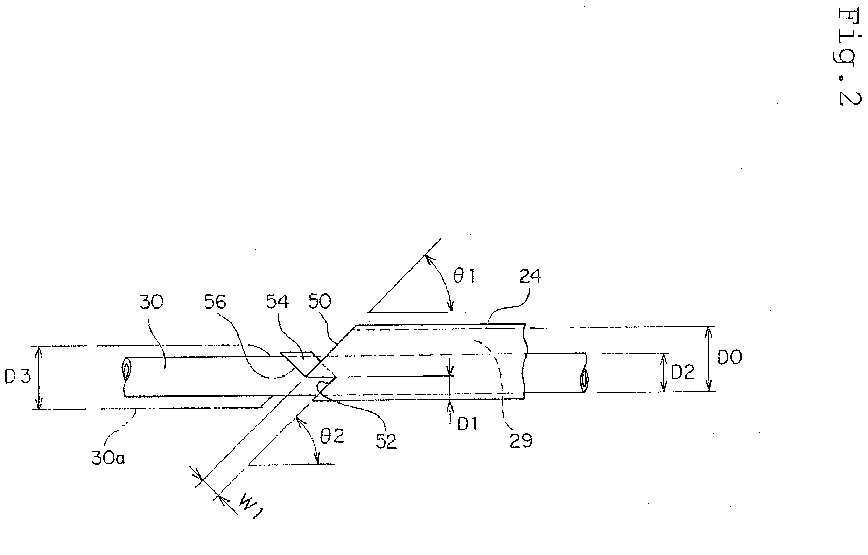

In the present embodiment, as shown in FIG. 2, the distal end of the outer tube 24 is formed with a cut 52 extending in the circumferential direction of the outer tube 24 at a position a predetermined width W1 away from the distal end opening face 50 toward the proximal end. The cut angle θ2 of the cut 52 is not particularly limited, but is preferably substantially the same as an angle θ1 of a later explained distal end opening face 50. Further, the cut 52 is preferably formed at a distance of a predetermined width W1 away from the edge of the most distal end at the distal end opening face 50 in the axial direction.

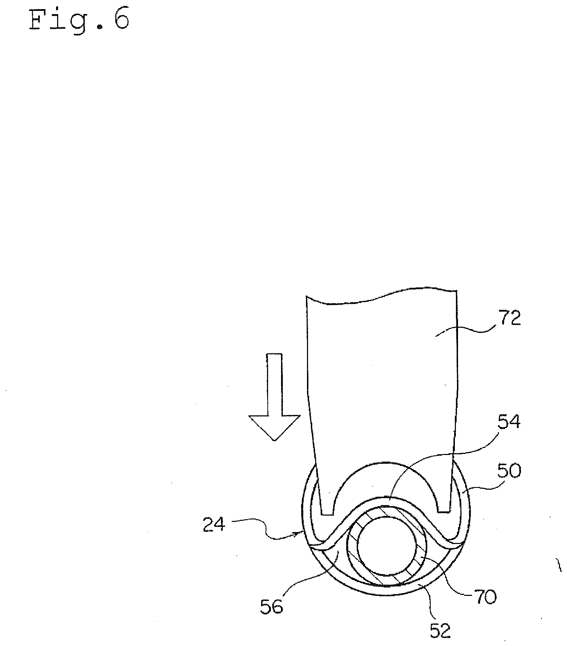

The depth D1 of the cut 52 in a perpendicular direction to the longitudinal axis of the outer tube 24 is preferably a depth of not more than the outside diameter D2 of the inner tube 30, more preferably 55 to 90% of the outside diameter D2. If this depth D1 is too small, formation of an engagement hole 56 for inserting the later explained inner tube 30 (see FIG. 6) becomes difficult, while if too large, engagement of the outer tube 24 and inner tube 30 may become insufficient.

The predetermined width W1 is not particularly limited, but is preferably 1 to 3 mm. If this width W1 is too small, engagement of inner tube 30 may become insufficient, while if too large, the width of the cut piece 54 formed by the cut 52 becomes large, so the cut piece 54 may enter the inlet/outlet port of the pressurized fluid conduit 29 at the distal end opening face 50, and the response of the balloon part 22 may deteriorate.

The cut piece 54, formed by the part of the wall of the outer tube 24 positioned from the cut 52 to the distal end opening face 50, is pushed into the outer tube 24 as shown in FIG. 2, FIG. 5, and FIG. 6. Due to this, the engagement hole 56 allowing insertion of the inner tube 30 is formed. By inserting the inner tube 30 into this engagement hole 56, the cut piece 54 acts as an engagement means. The inner tube 30 engages with the inside wall of the outer tube 24 at this cut piece.

In the balloon catheter 20 of the present embodiment, the inner tube 30 is fastened to the inside wall of the outer tube 24 by inserting the inner tube 30 in the engagement hole 56, so in comparison to fastening by only adhesion, it is harder for the inner tube 30 to peel apart from the outer tube 24. Also, the fastening means can be composed without inserting a separate member and by just providing a cut at the distal end of the outer tube, so the distal end opening face 50 in the outer tube 24 can be made broader and the response of the balloon part 22 will not deteriorate. Further, the engagement hole 56 is formed by just forming the cut 52 and pushing the cut 54 in, so the work is simple.

In the present embodiment, as shown in FIG. 1 and FIG. 2, at the distal end of the outer tube 24 at the outer circumference of which the proximal end part of the balloon part 22 is connected, the distal end opening face 50 of the outer tube 24 forms an acute angle with the longitudinal axis of the outer tube 24. In FIG. 2, the angle θ1 of the distal end opening face 50 with respect to the longitudinal axis of the outer tube 24 is preferably 20 to 80 degrees, further preferably 35 to 50 degrees. If this angle θ1 is too small, the distal end opening face 50 enters the inside of the balloon part 22 too much and is liable to inhibit the inflation and deflation operation of the balloon part 22. Further, if this angle θ1 is too large, the cut piece 54 may enter the inlet/outlet port of the pressurized fluid conduit 29 at the distal end opening face 50 and the response of the balloon part 22 may deteriorate.

Further, in the balloon catheter 20 of the present embodiment, the engagement hole 56 is formed so that the cut piece 54 protrudes out from the distal end opening face 50 of the outer tube 24 to the distal end side, so the cut piece 54 will no longer narrow the inlet/outlet port of the pressurized fluid conduit 29 at the distal end opening face 50 of the outer tube 24 and the flow resistance can be lowered. As a result, the response of the balloon part 22 is improved.

Note that the construction of using the cut piece 54 of the present embodiment to engage the inner tube 30 with the inside wall of the outer tube 24 exhibits the effect of strongly fastening the inner tube 30 and outer tube 24 near the distal end of the outer tube 24 by a simple means and increasing the opening area at the distal end of the outer tube 24. Therefore, this construction can also be applied to a balloon catheter where the outer tube and inner tube are not fastened at all or a balloon catheter where they are fastened by adhesion or fusion over a length of less than 50% of the total length of the outer tube. Further, a balloon catheter having a construction engaging the inner tube with the inside wall of the outer tube by this cut piece may be used as a balloon catheter other than an intra-aortic balloon catheter, for example, may be used as a PTCA catheter, PTA catheter, etc.

The outside diameter D2 of the inner tube 30 is not particularly limited, but is preferably 0.5 to 2 mm, more preferably 30 to 60% of the inside diameter DO of the outer tube 24. The outside diameter D2 of this inner tube 30 is the same along the axial direction in the present embodiment. The inner tube 30 may be composed of, for example, a polyurethane, polyvinyl chloride, polyethylene, nylon, polyether ether ketone, or other plastic tube or a nickel-titanium alloy thin tube, stainless steel thin tube, etc. Further, when the inner tube 30 is composed of a plastic tube, a stainless steel wire etc. may be embedded in it.

The outer tube 24 is not particularly limited, but may be composed of polyurethane, polyvinyl chloride, polyethylene terephthalate, polyamide, or another plastic and may have a stainless steel wire etc. embedded in it. The inside diameter D0 and thickness of the outer tube 24 are not particularly limited, but the inside diameter D0 is preferably 1.5 to 4.0 mm and the thickness is preferably 0.05 to 0.4 mm. The length of the outer tube 24 is preferably 300 to 800 mm.

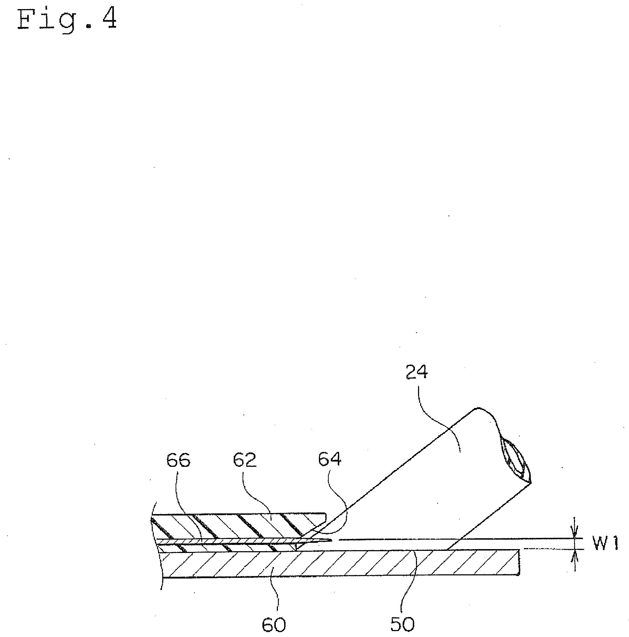

To form the cut 52 at the distal end of the outer tube 24, as shown in FIG. 4, the distal end of the outer tube 24 is cut at an angle, then the distal end opening face 50 is brought into contact with a slide 60 and made to move in the distal end direction of the blade 66. The blade 66 is held on a table top 62 so as to be at the position of a predetermined height W1 (same as predetermined width W1) from the surface of the slide 60. The front end of the table top 62 is formed with a recess 64. Inside that recess 64, the front end of the blade 66 protrudes by a predetermined length. The protruding length of this blade 66 determines the depth of the cut 52.

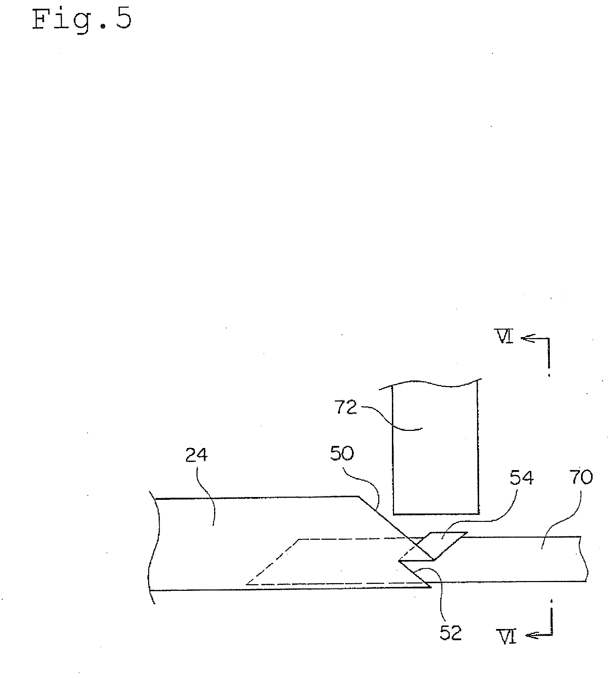

After this, as shown in FIG. 5 and FIG. 6, the cut piece 54 is pushed to the inside of the outer tube 24 to fold it back to make the cut piece 54 protrude from the distal end opening face 50 to the distal end side and shape the engagement hole 56. To shape the engagement hole 56, a mental mandrel 70 of an outside diameter the same as the outside diameter of the inner tube 30 or slightly smaller is passed through the engagement hole 56 and a shaping iron heated to for example about 90°C is pressed against the cut piece 54 from the outside. As a result, the shape of the engagement hole 56 is maintained.

After this, the mandrel 70 is removed from the engagement hole 56. In its place, the inner tube 30 is inserted. Next, as shown in FIG. 1 and FIG. 3, an adhesive 35 is used to adhere the inner tube 30 to the inside wall of the outer tube 24 along a predetermined length L1 from the distal end of the outer tube 24. Further, it is preferable to coat and cure the adhesive so as to surround the cut piece 54 and the part of the inner tube 30 positioned near it. By coating the adhesive in this way, breakage of the cut piece 54 is prevented.

After this, the distal end of the balloon part 22 is connected to the distal end of the inner tube 30 using adhesion etc., while the proximal end of the balloon part 22 is joined to the outer circumference of the distal end of the outer tube 24 by fusion etc. Further, the branching part 26 is connected by adhesion etc. to the proximal end of the outer tube 24.

In the balloon catheter according to the present embodiment, the outer tube 24 and the inner tube 30 are fastened along a predetermined length L1 and, further, at the distal end of the outer tube 24, the inner tube 30 is engaged by a cut piece 54. Due to this engagement, even if the intra-aortic balloon catheter 20 is driven for a long period, the inner tube 30 fastened by adhesion will no longer peel off from the outer tube 24 and the phenomenon of the response deteriorating over time will be prevented.

Further, in the present embodiment, by just forming a cut 52 at the distal end of the outer tube 24 and pushing in the obtained cut piece 54, an engagement means is formed, thus the work efficiency for engagement is improved and detachment of the engagement means can be avoided.

Note that in the present embodiment, the engagement means is not limited to the illustrated cut piece 54. It may also be another structure. For example, a tube member having a recess at its outer circumference along the axial direction may be inserted into the distal end of the outer tube 24 so that the inner tube 30 fits in the recess for engagement. As other examples of engagement means, there are ring members, U-shaped members, clips, string (knotting), etc.

### Second Embodiment

In the present embodiment, as shown in FIG. 2, the outside diameter D3 of the inner tube 30a positioned at the distal end side from the cut piece 54 is greater than the outside diameter D2 of the inner tube 30 positioned at the proximal end side from the cut piece 54. Conversely speaking, the outside diameter D2 of the inner tube 30 positioned at the proximal end side from the cut piece 54 is smaller than the outside diameter D3 of the inner tube 30a positioned at the distal end side from the cut piece 54. The rest of the configuration is the same as the first embodiment, so detailed explanations are omitted.

The inside diameter of the blood conduit 31 formed inside the inner tube 30 is preferably the same at the inner tube 30a of the distal end side and the inner tube 30 of the proximal end side, but does not have to necessarily be the same. In the case where the inside diameter of the blood conduit 31 is made the same at the inner tube 30a of the proximal end side and the inner tube 30 of the proximal end side, the thickness of inner tube 30 of the proximal end side may be reduced.

The outside diameter D3 of the inner tube 30a of the distal end side is preferably 1 to 30% larger than the outside diameter D2 of the inner tube 30 of the proximal end side. By forming it that way, even if the balloon part 22 is pushed back by the blood flow, the larger outside diameter inner tube 30a will be caught on the cut piece 54, so the balloon part 22 will be prevented from being pushed back further.

Further, in the present embodiment, inside the balloon part 22, by setting the outside diameter of the inner tube 30a at the distal end side about the same as the inner tube of a conventional balloon catheter and setting the outside diameter of the inner tube 30 of the proximal end side positioned inside the outer tube 24 smaller than the past, the following actions and effects are exhibited. In other words, it is possible to give the inner tube 30 positioned inside the balloon part 22 sufficient rigidity to support the balloon part 22 and to increase the cross-sectional area of the pressurized fluid conduit 29 to improve the response of the balloon part 22. Note that inside the outer tube 24, since the outer tube 24 itself has some rigidity, even if the rigidity of the inner tube 30 falls, there is no problem.

### Other Embodiments

Note that the present invention is not limited in scope to the above embodiments. Various modifications are possible within the scope of the invention.

For example, the shape of the illustrated distal end opening face 50 and the shape of the cut 52 are not limited to straight shapes and may be curved.

### (EXAMPLES)

Below, the present invention will be explained based on more detailed examples, but the present invention is not limited to these examples.

### Example 1

The balloon catheter 20 shown in FIG. 1 to FIG. 3 was manufactured. One, in FIG. 2, with an outside diameter D2 of the inner tube 30 of 1.10 mm, an inside diameter of 0.84 mm, and the same outside diameter along the axial direction was used. The outside diameter of the outer tube 24 was 2.70 mm, the inside diameter D0 was 2.40 mm, and the total length was 600 mm. The angle θ1 of the distal end opening face 50 of the tube 24 was 40 degrees. Further, the angle θ2 of the cut 52 was the same as the angle θ1, and the depth D1 of the cut 52 was 0.6 mm. The width of the cut piece 54 was 1.0 mm.

The material forming outer tube 24 was polyurethane, the material forming the inner tube 30 was polyether ether ketone, the material of the balloon film forming the tubular balloon part 22 was polyurethane, and the inside capacity of the balloon part 22 was 40 cc. The inner tube 30 was passed through the engagement hole 56 of the cut piece 54 and was fastened on a substantially straight line to the inside wall of the outer tube 24 by a cyanoacrylate-based adhesive over a predetermined length L1=500 mm (83% of total length of outer tube 24) at the proximal end side from the cut piece 54.

The results of experiments using this balloon catheter 20 to investigate the response of inflation and deflation of the balloon part 22 under the following conditions are shown next. As the pressurized fluid flowing in the pressurized fluid conduit 29, helium was used.

The outer tube of the balloon catheter was bent three times in succession in semi-circles with a curvature of approximately of a radius of 5 cm. In that state, helium was introduced and guided out at the balloon part through the inside of the outer tube (pressurized fluid conduit). The time T1 until maximum inflation from the state of maximum deflation of the balloon part and the time TD until maximum deflation from maximum inflation were investigated. These were measured five times. From the average, TI+TD is 203 ms at the initial drive period. After consecutive driving over the next 14 days, it was 204 ms, that is, it was confirmed that there was almost no change. Note that the smaller the TI+TD, the better the response.

### Example 2

Other than making the angle θ1 of the distal end opening face 50 shown in FIG. 2 and the angle θ2 of the cut 52 90 degrees, the same procedure was followed as in Example 1 to produce a balloon catheter. This was tested in the same way. TI+TD, at initial drive period, was 221 ms. After consecutive driving over the next 14 days, it was 222 ms, that is, it was confirmed that there was almost no change.

### Comparative Example 1

Other than not forming a cut 52 and not fastening the inner tube by an engagement hole 56, the same procedure was followed as in Example 1 to produce a balloon catheter. This was tested in the same way.

TI+TD, at the initial drive period, was 202 ms. After consecutive driving over the next 14 days, it was 232 ms, that is, it was confirmed that the response deteriorated over time.

### Comparative Example 2

Other than not forming a cut 52, not fastening the inner tube through an engagement hole 56, and not adhering the inner tube to the inside wall of the outer tube by an adhesive, the same procedure was followed as in Example 1 to produce a balloon catheter. This was tested in the same way.

TI+TD, at the initial drive period, was 268 ms. After consecutive driving over the next 14 days, it was 269 ms.

### Reference Example

Other than not adhering the inner tube to the inside wall of the outer tube by an adhesive, the same procedure was followed as in Example 1 to produce a balloon catheter. This was tested in the same way. TI+TD, at the initial drive period, was 252 ms. After consecutive driving over the next 14 days, it was 254 ms. Almost no deterioration of the response over time was seen. The response was inferior in comparison to Examples 1 and 2, but in comparison to Comparative Example 2, a sufficient improvement in response was confirmed. It was confirmed that this was of a practical level.

## Claims

1. An intra-aortic balloon catheter comprising;
a balloon part (22) inserted inside the aorta and inflating and deflating to assist the heart function,
an outer tube (24) with a distal end connected to a proximal end of said balloon part (22) to introduce and guide out pressurized fluid inside of said balloon part (22), and
an inner tube (30) to which a distal end of said balloon part (22) is connected and extending through the insides of said balloon part (22) and said outer tube (24) in the axial direction, wherein
said outer tube (24) and said inner tube (30) are fastened by adhesion or fusion over a length of 50% or greater of the total length from the distal end of the outer tube (24), and
said outer tube (24) and said inner tube (30) are further fastened by engagement with an engagement means (54) engaging said inner tube (30) with the inside wall of said outer tube (24) at the distal end of said outer tube (24).

2. The intra-aortic balloon catheter as set forth in claim 1, wherein
the distal end of said outer tube (24) is formed with a cut (52) that extends in the circumferential direction of said outer tube (24) at a predetermined distance away from the distal end opening face (50) of said outer tube (24) toward the proximal end,
an engagement hole (56) enabling insertion of said inner tube (30) is formed by part of the wall of said outer tube (24) positioned in the distance from said cut (52) to said distal end opening face (50), constituting a cut piece (54), being pushed in toward the inside of said outer tube (24), and
said inner tube (30) is inserted in said engagement hole (56) for engagement with the inside wall of said outer tube (24).

3. The intra-aortic balloon catheter as set forth in claim 2, wherein
the distal end opening face (50) of said outer tube (24) forms an acute angle (θ) with a longitudinal axis of said outer tube (24).

4. The intra-aortic balloon catheter as set forth in claim 3, wherein
said engagement hole (56) is formed with said cut piece (54) positioned so that it protrudes from the distal end opening face (50) of said outer tube (24) to the distal end side.

5. The intra-aortic balloon catheter as set forth in claim 1, wherein the outside diameter (D3) of said inner tube (30a) positioned at the distal end side from said engagement means (54) is larger than the outside diameter (D2) of said inner tube (30) positioned at the proximal end side from said engagement means (54).

## Patentansprüche

1. Intra-aortaler Ballonkatheter umfassend;
einen Ballonteil (22), der in das Innere der Aorta eingeführt wird und aufgeblasen und entleert wird, um der Herzfunktion zu assistieren,
eine äußere Röhre (24) mit einem distalen Ende, das mit einem proximalen Ende des Ballonteils (22) verbunden ist, um ein unter Druck stehendes Fluid in das Innere des Ballonteils (22) einzuführen und auszuleiten, und
eine innere Röhre (30), mit der ein distales Ende des Ballonteils (22) verbunden ist, und die sich durch das Innere des Ballonteils (22) und der äußeren Röhre (24) in die axiale Richtung erstreckt, wobei
die äußere Röhre (24) und die innere Röhre (30) durch Adhäsion oder Verschmelzung über eine Länge von 50% oder größer von der Gesamtlänge von dem distalen Ende der äußeren Röhre (24) aus befestigt sind, und
die äußere Röhre (24) und die innere Röhre (30) ferner durch Bindung mit einem Bindungsmittel (54), das die innere Röhre (30) mit der inneren Wand der äußeren Röhre (24) an dem distalen Ende der äußeren Röhre (24) verbindet, befestigt sind.

2. Intra-aortaler Ballonkatheter nach Anspruch 1, wobei
das distale Ende der äußeren Röhre (24) mit einem Schnitt (52) gebildet wird, welcher sich in die Umfangsrichtung der äußeren Röhre (24) bei einem vordefinierten Abstand weg von einer Öffnungsfläche (50) der äußeren Röhre (24) auf das proximale Ende zu erstreckt,
ein Bindungsloch (56), das eine Einführung der inneren Röhre (30) ermöglicht, durch einen Teil der Wand der äußeren Röhre (24), die in dem Abstand des Schnittes (52) zum distalen Ende der Öffnungsfläche (50) angeordnet ist, gebildet wird, ein Schnittteil (54) bildet, das auf das Innere der äußeren Röhre (24) gedrückt wird, und die innere Röhre (30) in das Bindungsloch (56) für die Bindung mit der inneren Wand der äußeren Röhre (24) eingeführt wird.

3. Intra-aortaler Ballonkatheter nach Anspruch 2, wobei
die Öffnungsfläche (50) des distalen Endes der äußeren Röhre (24) einen akuten Winkel (θ) mit einer longitudinalen Achse der äußeren Röhre (24) bildet.

4. Intra-aortaler Ballonkatheter nach Anspruch 3, wobei
das Bindungsloch (56), das mit dem Schnittteil (54) gebildet wird, derart angeordnet ist, dass es aus der Öffnungsfläche (50) des distalen Endes der äußeren Röhre (24) zur Seite des distalen Endes herausragt.

5. Intra-aortaler Ballonkatheter nach Anspruch 1, wobei der Außendurchmesser (D3) der inneren Röhre (30a), der auf der Seite des distalen Endes des Bindungsmittels (54) angeordnet ist, größer ist als der Außendurchmesser (D2) der inneren Röhre (30), die auf der Seite des proximalen Endes des Bindungsmittels (54) angeordnet ist.

## Revendications

1. Cathéter à ballonnet intra-aortique comportant :
une partie de ballonnet (22) insérée à l'intérieur de l'aorte et se gonflant et se dégonflant pour assister la fonction cardiaque,
un tube extérieur (24) ayant une extrémité distale raccordée à une extrémité proximale de ladite partie de ballonnet (22) pour introduire et guider vers l'extérieur du fluide mis sous pression à l'intérieur de ladite partie de ballonnet (22), et
un tube intérieur (30) auquel une extrémité distale de ladite partie de ballonnet (22) est raccordée et s'étendant à travers les intérieurs de ladite partie de ballonnet (22) et dudit tube extérieur (24) dans la direction axiale, dans lequel
ledit tube extérieur (24) et ledit tube intérieur (30) sont fixés par adhérence ou fusion sur une longueur de 50 % ou plus de la longueur totale à partir de l'extrémité distale du tube extérieur (24), et
ledit tube extérieur (24) et ledit tube intérieur (30) sont en outre fixés par engagement avec des moyens d'engagement (54) engageant ledit tube intérieur (30) avec la paroi intérieure dudit tube extérieur (24) à l'extrémité distale dudit tube extérieur (24) .

2. Cathéter à ballonnet intra-aortique selon la revendication 1, dans lequel
l'extrémité distale dudit tube extérieur (24) est formée avec une découpe (52) qui s'étend dans la direction circonférentielle dudit tube extérieur (24) à une distance prédéterminée en s'écartant de la face d'ouverture d'extrémité distale (50) dudit tube extérieur (24) vers l'extrémité proximale,
un trou d'engagement (56) permettant une insertion dudit tube intérieur (30) est formé par une partie de la paroi dudit tube extérieur (24) positionnée dans la distance à partir de ladite découpe (52) jusqu'à ladite face d'ouverture d'extrémité distale (50), constituant une pièce coupée (54), étant poussée vers l'intérieur dudit tube extérieur (24), et
ledit tube intérieur (30) est inséré dans ledit trou d'engagement (56) en vue d'un engagement avec la paroi intérieure dudit tube extérieur (24).

3. Cathéter à ballonnet intra-aortique selon la revendication 2, dans lequel
la face d'ouverture d'extrémité distale (50) dudit tube extérieur (24) forme un angle aigu (θ) avec un axe longitudinal dudit tube extérieur (24).

4. Cathéter à ballonnet intra-aortique selon la revendication 3, dans lequel
ledit trou d'engagement (56) est formé avec ladite pièce coupée (54) positionnée de sorte qu'elle fait saillie à partir de la face d'ouverture d'extrémité distale (50) dudit tube extérieur (24) jusqu'au côté d'extrémité distale.

5. Cathéter à ballonnet intra-aortique selon la revendication 1, dans lequel le diamètre extérieur (D3) dudit tube intérieur (30a) positionné sur le côté d'extrémité distale par rapport auxdits moyens d'engagement (54) est plus grand que le diamètre extérieur (D2) dudit tube intérieur (30) positionné sur le côté d'extrémité proximale par rapport auxdits moyens de prise (54).
